# EUROPEAN PATENT SPECIFICATION

(11) **EP 3 802 784 B1**
(45) Date of publication and mention of the grant of the patent: **07.08.2024**
(21) Application number: 19731823.1
(22) Date of filing: 21.05.2019
(51) Int. Cl.: C12N 1/18, C12N 1/16, C12P 7/06, C07K 14/395

(54) **OVER-EXPRESSION OF TRANSCRIPTIONAL ACTIVATOR/REPRESSOR GIS1 IN YEAST FOR INCREASED ETHANOL PRODUCTION**
ÜBEREXPRESSION VON TRANSKRIPTIONSAKTIVATOR/REPRESSOR GIS1 IN HEFE FÜR ERHÖHTE ETHANOLPRODUKTION
SUREXPRESSION DE L'ACTIVATEUR/RÉPRESSEUR TRANSCRIPTIONNEL GIS1 DANS LA LEVURE POUR UNE PRODUCTION ACCRUE D'ÉTHANOL

(30) Priority: 30.05.2018 US 201862677888 P
(43) Date of publication of application: 14.04.2021
(73) Proprietor: Danisco US Inc., Palo Alto, CA 94304 (US)
(72) Inventor: MACOOL, Daniel, Joseph, Rutledge, PA 19070 (US); TEUNISSEN, Paula, Johanna, Maria, Palo Alto, CA 94304 (US); ZHU, Quinn Qun, West Chester, PA 19382 (US)
(74) Representative: Mewburn Ellis LLP
(86) International application number: PCT/US2019/033207
(87) International publication number: WO 2019/231743

(56) References cited:
- WO-A1-2009/056984
- WO-A1-2015/148272
- YAO YU ET AL: "The JmjC domain of Gis1 is dispensable for transcriptional activation : Gis JmjC domain dispensable for transcriptional activation", FEMS YEAST RESEARCH, vol. 10, no. 7, 1 November 2010 (2010-11-01), GB, NL, pages 793 - 801, XP055607484, ISSN: 1567-1356, DOI: 10.1111/j.1567-1364.2010.00680.x
- I. PEDRUZZI: "Saccharomyces cerevisiae Ras/cAMP pathway controls post-diauxic shift element-dependent transcription through the zinc finger protein Gis1", THE EMBO JOURNAL, vol. 19, no. 11, 1 June 2000 (2000-06-01), pages 2569 - 2579, XP055607488, ISSN: 0261-4189, DOI: 10.1093/emboj/19.11.2569

## Description

### TECHNICAL FIELD

The present compositions and methods relate to modified yeast cells that over-expresses the transcriptional activator/repressor, GIS 1. The yeast cells produce an increased amount of ethanol compared to their parental cells. Such yeast is particularly useful for large-scale ethanol production from starch substrates where increased ethanol production is desirable.

### BACKGROUND

Yeast-based ethanol production is based on the conversion of sugars into ethanol. The current annual fuel ethanol production by this method is about 90 billion liters worldwide. It is estimated that about 70% of the cost of ethanol production is the feedstock. Since the ethanol production volume is so large, even small yield improvements have massive economic impact for the industry. The conversion of one mole of glucose into two moles of ethanol and two moles of carbon dioxide is redox-neutral, with the maximum theoretical yield being about 51%. The current industrial yield is about 45%; therefore, there are opportunities to increase ethanol production. Despite advances in yeast productivity, the need exists to further modify yeast metabolic pathways to maximize ethanol production, while not increasing the production of undesirable by-products.

### SUMMARY

The present invention, as defined in the appended claims, concerns a method for increasing the production of ethanol in yeast cells grown on a carbohydrate substrate, comprising: introducing into parental yeast cells a genetic alteration that causes the modified cells to produce an increased amount of GIS 1 polypeptides compared to the parental cells, wherein the modified cells produce an increased amount of ethanol compared to otherwise identical parental cells under equivalent fermentation conditions.

### DETAILED DESCRIPTION

### I. Definitions

Prior to describing the present yeast and methods in detail, the following terms are defined for clarity. Terms not defined should be accorded their ordinary meanings as used in the relevant art.

As used herein, the term "alcohol" refers to an organic compound in which a hydroxyl functional group (-OH) is bound to a saturated carbon atom.

As used herein, the terms "yeast cells," "yeast strains," or simply "yeast" refer to organisms from the phyla Ascomycota and Basidiomycota. Exemplary yeast is budding yeast from the order Saccharomycetales. Particular examples of yeast are *Saccharomyces* spp., including but not limited to *S. cerevisiae.* Yeast include organisms used for the production of fuel alcohol as well as organisms used for the production of potable alcohol, including specialty and proprietary yeast strains used to make distinctive-tasting beers, wines, and other fermented beverages.

As used herein, the phrase "engineered yeast cells," "variant yeast cells," "modified yeast cells," or similar phrases, refer to yeast that include genetic modifications and characteristics described herein. Variant/modified yeast do not include naturally occurring yeast.

As used herein, the terms "polypeptide" and "protein" (and their respective plural forms) are used interchangeably to refer to polymers of any length comprising amino acid residues linked by peptide bonds. The conventional one-letter or three-letter codes for amino acid residues are used herein and all sequence are presented from an N-terminal to C-terminal direction. The polymer can comprise modified amino acids, and it can be interrupted by non-amino acids. The terms also encompass an amino acid polymer that has been modified naturally or by intervention; for example, disulfide bond formation, glycosylation, lipidation, acetylation, phosphorylation, or any other manipulation or modification, such as conjugation with a labeling component. Also included within the definition are, for example, polypeptides containing one or more analogs of an amino acid (including, for example, unnatural amino acids, etc.), as well as other modifications known in the art.

As used herein, functionally and/or structurally similar proteins are considered to be "related proteins," or "homologs." Such proteins can be derived from organisms of different genera and/or species, or different classes of organisms (e.g., bacteria and fungi), or artificially designed. Related proteins also encompass homologs determined by primary sequence analysis, determined by secondary or tertiary structure analysis, or determined by immunological cross-reactivity, or determined by their functions.

As used herein, the term "homologous protein" refers to a protein that has similar activity and/or structure to a reference protein. It is not intended that homologs necessarily be evolutionarily related. Thus, it is intended that the term encompass the same, similar, or corresponding enzyme(s) (i.e., in terms of structure and function) obtained from different organisms. In some embodiments, it is desirable to identify a homolog that has a quaternary, tertiary and/or primary structure similar to the reference protein. In some embodiments, homologous proteins induce similar immunological response(s) as a reference protein. In some embodiments, homologous proteins are engineered to produce enzymes with desired activity(ies).

The degree of homology between sequences can be determined using any suitable method known in the art (see, *e.g.,* Smith and Waterman (1981) Adv. Appl. Math. 2:482; Needleman and Wunsch (1970) J. Mol. Biol., 48:443; Pearson and Lipman (1988) Proc. Natl. Acad. Sci. USA 85:2444; programs such as GAP, BESTFIT, FASTA, and TFASTA in the Wisconsin Genetics Software Package (Genetics Computer Group, Madison, WI); and Devereux et al. (1984) Nucleic Acids Res. 12:387-95).

For example, PILEUP is a useful program to determine sequence homology levels. PILEUP creates a multiple sequence alignment from a group of related sequences using progressive, pair-wise alignments. It can also plot a tree showing the clustering relationships used to create the alignment. PILEUP uses a simplification of the progressive alignment method of Feng and Doolittle, (Feng and Doolittle (1987) J. Mol. Evol. 35:351-60). The method is similar to that described by Higgins and Sharp ((1989) CABIOS 5:151-53). Useful PILEUP parameters including a default gap weight of 3.00, a default gap length weight of 0.10, and weighted end gaps. Another example of a useful algorithm is the BLAST algorithm, described by Altschul et al. ((1990) J. Mol. Biol. 215:403-10) and Karlin et al. ((1993) Proc. Natl. Acad. Sci. USA 90:5873-87). One particularly useful BLAST program is the WU-BLAST-2 program (see, *e.g.,* Altschul et al. (1996) Meth. Enzymol. 266:460-80). Parameters "W," "T," and "X" determine the sensitivity and speed of the alignment. The BLAST program uses as defaults a word-length (W) of 11, the BLOSUM62 scoring matrix (see, *e.g.,* Henikoff and Henikoff (1989) Proc. Natl. Acad. Sci. USA 89:10915) alignments (B) of 50, expectation (E) of 10, M'5, N'-4, and a comparison of both strands.

As used herein, the phrases "substantially similar" and "substantially identical," in the context of at least two nucleic acids or polypeptides, typically means that a polynucleotide or polypeptide comprises a sequence that has at least about 70% identity, at least about 75% identity, at least about 80% identity, at least about 85% identity, at least about 90% identity, at least about 91% identity, at least about 92% identity, at least about 93% identity, at least about 94% identity, at least about 95% identity, at least about 96% identity, at least about 97% identity, at least about 98% identity, or even at least about 99% identity, or more, compared to the reference (*i.e.*, wild-type) sequence. Percent sequence identity is calculated using CLUSTAL W algorithm with default parameters. See Thompson et al. (1994) Nucleic Acids Res. 22:4673-4680. Default parameters for the CLUSTAL W algorithm are:

| | |
|---|---|
| Gap opening penalty: | 10.0 |
| Gap extension penalty: | 0.05 |
| Protein weight matrix: | BLOSUM series |
| DNA weight matrix: | IUB |
| Delay divergent sequences %: | 40 |
| Gap separation distance: | 8 |
| DNA transitions weight: | 0.50 |
| List hydrophilic residues: | GPSNDQEKR |
| Use negative matrix: | OFF |
| Toggle Residue specific penalties: | ON |
| Toggle hydrophilic penalties: | ON |
| Toggle end gap separation penalty | OFF |

Another indication that two polypeptides are substantially identical is that the first polypeptide is immunologically cross-reactive with the second polypeptide. Typically, polypeptides that differ by conservative amino acid substitutions are immunologically cross-reactive. Thus, a polypeptide is substantially identical to a second polypeptide, for example, where the two peptides differ only by a conservative substitution. Another indication that two nucleic acid sequences are substantially identical is that the two molecules hybridize to each other under stringent conditions (*e.g*., within a range of medium to high stringency).

As used herein, the term "gene" is synonymous with the term "allele" in referring to a nucleic acid that encodes and directs the expression of a protein or RNA. Vegetative forms of filamentous fungi are generally haploid, therefore a single copy of a specified gene (*i.e.,* a single allele) is sufficient to confer a specified phenotype. The term "allele" is generally preferred when an organism contains more than one similar genes, in which case each different similar gene is referred to as a distinct "allele."

As used herein, the term "expressing a polypeptide" and similar terms refers to the cellular process of producing a polypeptide using the translation machinery (*e.g.*, ribosomes) of the cell.

As used herein, "over-expressing a polypeptide," "increasing the expression of a polypeptide," and similar terms, refer to expressing a polypeptide at higher-than-normal levels compared to those observed with parental or "wild-type cells that do not include a specified genetic modification. Overexpression may be described in terms of mRNA levels or polypeptide levels, as experimental conditions permit.

As used herein, an "expression cassette" refers to a DNA fragment that includes a promoter, and amino acid coding region and a terminator (*i.e.*, promoter: :amino acid coding region: terminator) and other nucleic acid sequence needed to allow the encoded polypeptide to be produced in a cell. Expression cassettes can be exogenous (*i.e.*, introduced into a cell) or endogenous (*i.e.*, extant in a cell).

As used herein, the terms "fused" and "fusion" with respect to two DNA fragments, such as a promoter and the coding region of a polypeptide refer to a physical linkage causing the two DNA fragments to become a single molecule.

As used herein, the terms "wild-type" and "native" are used interchangeably and refer to genes, proteins or strains found in nature, or that are not intentionally modified for the advantage of the presently described yeast.

As used herein, the term "protein of interest" refers to a polypeptide that is desired to be expressed in modified yeast. Such a protein can be an enzyme, a substrate-binding protein, a surface-active protein, a structural protein, a selectable marker, a signal transducer, a receptor, a transporter, a transcription factor, a translation factor, a co-factor, or the like, and can be expressed. The protein of interest is encoded by an endogenous gene or a heterologous gene (*i.e.,* gene of interest") relative to the parental strain. The protein of interest can be expressed intracellularly or as a secreted protein.

As used herein, "disruption of a gene" refers broadly to any genetic or chemical manipulation, *i.e.,* mutation, that substantially prevents a cell from producing a function gene product, *e.g*., a protein, in a host cell. Exemplary methods of disruption include complete or partial deletion of any portion of a gene, including a polypeptide-coding sequence, a promoter, an enhancer, or another regulatory element, or mutagenesis of the same, where mutagenesis encompasses substitutions, insertions, deletions, inversions, and combinations and variations, thereof, any of which mutations substantially prevent the production of a function gene product. A gene can also be disrupted using CRISPR, RNAi, antisense, or any other method that abolishes gene expression. A gene can be disrupted by deletion or genetic manipulation of non-adj acent control elements. As used herein, "deletion of a gene," refers to its removal from the genome of a host cell. Where a gene includes control elements (*e.g.*, enhancer elements) that are not located immediately adjacent to the coding sequence of a gene, deletion of a gene refers to the deletion of the coding sequence, and optionally adjacent enhancer elements, including but not limited to, for example, promoter and/or terminator sequences, but does not require the deletion of non-adj acent control elements. Deletion of a gene also refers to the deletion a part of the coding sequence, or a part of promoter immediately or not immediately adjacent to the coding sequence, where there is no functional activity of the interested gene existed in the engineered cell.

As used herein, the terms "genetic manipulation" and "genetic alteration" are used interchangeably and refer to the alteration/change of a nucleic acid sequence. The alteration can include but is not limited to a substitution, deletion, insertion or chemical modification of at least one nucleic acid in the nucleic acid sequence.

As used herein, a "functional polypeptide/protein" is a protein that possesses an activity, such as an enzymatic activity, a binding activity, a surface-active property, a signal transducer, a receptor, a transporter, a transcription factor, a translation factor, a co-factor, or the like, and which has not been mutagenized, truncated, or otherwise modified to abolish or reduce that activity. Functional polypeptides can be thermostable or thermolabile, as specified.

As used herein, "a functional gene" is a gene capable of being used by cellular components to produce an active gene product, typically a protein. Functional genes are the antithesis of disrupted genes, which are modified such that they cannot be used by cellular components to produce an active gene product, or have a reduced ability to be used by cellular components to produce an active gene product.

As used herein, yeast cells have been "modified to prevent the production of a specified protein" if they have been genetically or chemically altered to prevent the production of a functional protein/polypeptide that exhibits an activity characteristic of the wild-type protein. Such modifications include, but are not limited to, deletion or disruption of the gene encoding the protein (as described, herein), modification of the gene such that the encoded polypeptide lacks the aforementioned activity, modification of the gene to affect post-translational processing or stability, and combinations, thereof.

As used herein, "attenuation of a pathway" or "attenuation of the flux through a pathway," *i.e.,* a biochemical pathway, refers broadly to any genetic or chemical manipulation that reduces or completely stops the flux of biochemical substrates or intermediates through a metabolic pathway. Attenuation of a pathway may be achieved by a variety of well-known methods. Such methods include but are not limited to: complete or partial deletion of one or more genes, replacing wild-type alleles of these genes with mutant forms encoding enzymes with reduced catalytic activity or increased Km values, modifying the promoters or other regulatory elements that control the expression of one or more genes, engineering the enzymes or the mRNA encoding these enzymes for a decreased stability, misdirecting enzymes to cellular compartments where they are less likely to interact with substrate and intermediates, the use of interfering RNA, and the like.

As used herein, "aerobic fermentation" refers to growth in the presence of oxygen.

As used herein, "anaerobic fermentation" refers to growth in the absence of oxygen.

As used herein, the expression "end of fermentation" refers to the stage of fermentation when the economic advantage of continuing fermentation to produce a small amount of additional alcohol is exceeded by the cost of continuing fermentation in terms of fixed and variable costs. In a more general sense, "end of fermentation" refers to the point where a fermentation will no longer produce a significant amount of additional alcohol, *i.e.,* no more than about 1% additional alcohol, or no more substrate left for further alcohol production.

As used, herein, a "glucoamylase unit (GAU)" is defined as the amount of glucoamylase required to produce 1 g of glucose per hour from soluble starch substrate (4% ds) under assay conditions of 60°C and pH 4.2.

As used herein, a "spectrophotometric acid protease unit (SAPU)" is the amount of protease activity that liberates one micromole of tyrosine per minute from a casein substrate under standard assay conditions.

As used herein, a "soluble starch unit (SSU)" is based on the degree of hydrolysis of soluble potato starch substrate (4% DS) by an aliquot of α-amylase at pH 4.5, 50°C. The reducing sugar content is measured using the DNS method as described by Miller, G.L. ((1959) Anal. Chem. 31:426-28).

As used herein, the singular articles "a," "an" and "the" encompass the plural referents unless the context clearly dictates otherwise. The following abbreviations/acronyms have the following meanings unless otherwise specified:
- °C: degrees Centigrade
- AA: α-amylase
- AADH: acetaldehyde dehydrogenases
- ADH: alcohol dehydrogenase
- bp: base pairs
- DNA: deoxyribonucleic acid
- ds or DS: dry solids
- EC: enzyme commission
- EtOH: ethanol
- g or gm: gram
- g/L: grams per liter
- GA: glucoamylase
- GAU: glucoamylase unit
- HPLC: high performance liquid chromatography
- hr or h: hour
- kg: kilogram
- M: molar
- mg: milligram
- min: minute
- mL or ml: milliliter
- mM: millimolar
- mRNA: messenger RNA
- N: normal
- nm: nanometer
- PCR: polymerase chain reaction
- PKL: phosphoketolase
- ppm: parts per million
- PTA: phosphotransacetylase
- SAPU: spectrophotometric acid protease unit
- SSU: soluble starch unit
- Δ: relating to a deletion
- µg: microgram
- µL and µl: microliter
- µM: micromolar

### II. Overexpression of GIS1 in yeast increases ethanol production in yeast

GIS1 is a transcription factor involved in the regulation of gene expression upon nutrient starvation. It recognizes and binds to the post-diauxic-shift element 5'-T[AT]AGGGAT-3' (SEQ ID NO: 1) in the promoter region. It can act as a transcriptional activator (*e.g.,* of stress genes like SSA3, HSP12 and HSP26) as well as a repressor (*e.g.,* of pyrophosphate phosphatase DPP1). GIS1 also acts as a DNA damage-responsive transcriptional repressor of photolyase PHR1. GIS1 and RPH1 have also been found to control a number of genes involved in acetate and glycerol formation, metabolites that have been implicated in aging.

It has now been discovered that GIS1 over-expression can increase ethanol production by more than 1% in non-genetically engineered yeast as well as engineered yeast harboring a genetically engineered pathway. RNAseq analyses suggest that GIS1 gene is normally weakly expressed. 10-fold over-expression of GIS1 increases ethanol production in otherwise identical yeast by at least 1%. The exemplified GIS1 polypeptide is from *Saccharomyces cerevisiae* S288c (Genbank Accession No. NP_010381.1; SEQ ID NO: 5; *infra).* Genbank lists numerous other polypeptide sequences with a high percentage of amino acid sequence identity to SEQ ID NO: 5, followed by a large number of other similar polypeptides. Most, if not all, of these similar polypeptides are expected to produce similar results in yeast cells.

In particular embodiments of the present compositions and methods, the amino acid sequence of the GIS1 polypeptide that is over-expressed in modified yeast cells has at least about 70%, at least about 75%, at least about 80%, at least about 85%, at least about 90%, at least about 91%, at least about 92%, at least about 93%, at least about 94%, at least about 95%, at least about 96%, at least about 97%, at least about 98%, or even at least about 99% identity, to SEQ ID NO: 5.

The increase in the amount of GIS1 produced by the modified cells may be an increase of at least 20%, at least 30%, at least 40%, at least 50%, at least 70%, at least 100%, at least 150%, at least 200%, at least 500%, at least 1,000%, or more, compared to the amount of GIS1 produced by parental cells grown under the same conditions. Alternatively, or additionally, the increase in the amount of GIS1 produced by the modified cells may be at least 10-fold, at least 12 fold, or at least 15 fold, or at least 20-fold, or more, compared to the amount of GIS1 produced by parental cells grown under the same conditions.

The increase in the strength of the promoter used to control expression of the GIS1 produced by the modified cells may be at least 10-fold, at least 12 fold, at least 15 fold, or at least 20-fold, or more, compared to strength of the native promoter controlling GIS 1 expression, based on the amount of mRNA produced.

It is understood that relative promoter strength is not an exact scalar value. It can strongly depend on culture medium, fermentation time, temperature and other conditions. Values obtained from RNAseq data collected over the time course of fermentation in industrial medium are the most preferred, however, experimental and/or literature data obtained under different cultivation may also be used for recombinant promoter selection.

Preferably, increased GIS1 expression is achieved by genetic manipulation using sequence-specific molecular biology techniques, as opposed to chemical mutagenesis, which is generally not targeted to specific nucleic acid sequences. However, chemical mutagenesis is not excluded as a method for making modified yeast cells.

In some embodiments, the present compositions and methods involve introducing into yeast cells a nucleic acid capable of directing the over-expression, or increased expression, of GIS1 polypeptide. Particular methods include but are not limited to (i) introducing an exogenous expression cassette for producing the polypeptide into a host cell, optionally in addition to an endogenous expression cassette, (ii) substituting an exogenous expression cassette with an endogenous cassette that allows the production of an increased amount of the polypeptide, (iii) modifying the promoter of an endogenous expression cassette to increase expression, (iv) increase copy number of the same or different cassettes for over-expression of GIS1 polypeptides, and/or (v) modifying any aspect of the host cell to increase the half-life of the polypeptide in the host cell.

In some embodiments, the parental cell that is modified already includes an engineered pathway of interest, such as a PKL pathway to increases ethanol production, or any other pathway to increase alcohol production. In some embodiments, the parental cell that is modified already includes a gene of interest, such as a gene encoding a selectable marker, carbohydrate-processing enzyme, or other polypeptide. In some embodiments, a gene of interest is subsequently introduced into the modified cells.

### III. Modified yeast cells overexpressing GIS1 and harboring an exogenous PKL pathway

Increased expression of GIS1 can be combined with expression of genes of the PKL pathway to further increase the production rate, and/or the maximum production levels, of ethanol in yeast cells.

Engineered yeast cells having a heterologous PKL pathway have been previously described in WO2015148272 (Miasnikov et al.)*.* These cells express heterologous phosphoketolase (PKL), phosphotransacetylase (PTA) and acetylating acetyl dehydrogenase (AADH), optionally with other enzymes, to channel carbon flux away from the glycerol pathway and toward the synthesis of acetyl-CoA, which is then converted to ethanol. Such modified cells are capable of increased ethanol production in a fermentation process when compared to otherwise-identical parent yeast cells.

### IV. Overexpressing of GIS1 in combination with other mutations that affect ethanol production

In some embodiments, in addition to overexpressing GIS1, optionally in combination with the presence of an exogenous PKL pathway, the present modified yeast cells include additional modifications that affect ethanol production.

The modified cells may further include mutations that result in attenuation of the native glycerol biosynthesis pathway and/or reuse glycerol pathway, which are known to increase alcohol production. Methods for attenuation of the glycerol biosynthesis pathway in yeast are known and include reduction or elimination of endogenous NAD-dependent glycerol 3-phosphate dehydrogenase (GPD) or glycerol phosphate phosphatase activity (GPP), for example by disruption of one or more of the genes *GPD*1*, GPD2, GPP1* and/or *GPP2.* See, *e.g.,* U.S. Patent Nos. 9,175,270 (Elke et al.)*,* 8,795,998 (Pronk et al.) and 8,956,851 (Argyros et al.)*.* Methods to enhance the reuse glycerol pathway by over expression of glycerol dehydrogenase (GCY1) and dihydroxyacetone kinase (DAK1) to convert glycerol to dihydroxyacetone phosphate (Zhang et al. (2013) J. Ind. Microbiol. Biotechnol. 40:1153-1160).

The modified yeast may further feature increased acetyl-CoA synthase (also referred to acetyl-CoA ligase) activity (EC 6.2.1.1) to scavenge (*i.e.,* capture) acetate produced by chemical or enzymatic hydrolysis of acetyl-phosphate (or present in the culture medium of the yeast for any other reason) and converts it to Ac-CoA. This partially reduces the undesirable effect of acetate on the growth of yeast cells and may further contribute to an improvement in alcohol yield. Increasing acetyl-CoA synthase activity may be accomplished by introducing a heterologous acetyl-CoA synthase gene into cells, increasing the expression of an endogenous acetyl-CoA synthase gene and the like.

In some embodiments the modified cells may further include a heterologous gene encoding a protein with NAD⁺-dependent acetylating acetaldehyde dehydrogenase (AADH) activity and/or a heterologous gene encoding a pyruvate formate lyase (PFL). The introduction of such genes in combination with attenuation of the glycerol pathway is described, *e.g.,* in U.S. Patent No. 8,795,998 (Pronk et al.)*.* In some embodiments of the present compositions and methods the yeast expressly lacks a heterologous gene(s) encoding an acetylating acetaldehyde dehydrogenase, a pyruvate formate lyase or both.

In some embodiments, the present modified yeast cells may further overexpress a sugar transporter-like (STL1) polypeptide to increase the uptake of glycerol (see, *e.g.,* Ferreira et al. (2005) Mol Biol Cell 16:2068-76; Dušková *et al.* (2015) *Mol Microbiol* 97:541-59 and WO 2015023989 A1).

In some embodiments, the present modified yeast cells further include a butanol biosynthetic pathway. In some embodiments, the butanol biosynthetic pathway is an isobutanol biosynthetic pathway. In some embodiments, the isobutanol biosynthetic pathway comprises a polynucleotide encoding a polypeptide that catalyzes a substrate to product conversion selected from the group consisting of: (a) pyruvate to acetolactate; (b) acetolactate to 2,3-dihydroxyisovalerate; (c) 2,3-dihydroxyisovalerate to 2-ketoisovalerate; (d) 2-ketoisovalerate to isobutyraldehyde; and (e) isobutyraldehyde to isobutanol. In some embodiments, the isobutanol biosynthetic pathway comprises polynucleotides encoding polypeptides having acetolactate synthase, keto acid reductoisomerase, dihydroxy acid dehydratase, ketoisovalerate decarboxylase, and alcohol dehydrogenase activity.

In some embodiments, the modified yeast cells comprising a butanol biosynthetic pathway further comprise a modification in a polynucleotide encoding a polypeptide having pyruvate decarboxylase activity. In some embodiments, the yeast cells comprise a deletion, mutation, and/or substitution in an endogenous polynucleotide encoding a polypeptide having pyruvate decarboxylase activity. In some embodiments, the polypeptide having pyruvate decarboxylase activity is selected from the group consisting of: PDC1, PDC5, PDC6, and combinations thereof. In some embodiments, the yeast cells further comprise a deletion, mutation, and/or substitution in one or more endogenous polynucleotides encoding FRA2, ALD6, ADH1, GPD2, BDH1, and YMR226C.

### V. GIS1 overexpression in combination with other beneficial mutations

In some embodiments, in addition to overexpression of GIS1, optionally in combination with other genetic modifications that benefit alcohol production, the present modified yeast cells further include any number of additional genes of interest encoding proteins of interest, or of reducing the production, thereof. Additional genes of interest may be introduced before, during, or after genetic manipulations that result in the overexpression of GIS 1 polypeptides. Proteins of interest, include selectable markers, carbohydrate-processing enzymes, and other commercially-relevant polypeptides, including but not limited to an enzyme selected from the group consisting of a dehydrogenase, a transketolase, a phosphoketolase, a transladolase, an epimerase, a phytase, a xylanase, a β-glucanase, a phosphatase, a protease, an α-amylase, a β-amylase, a glucoamylase, a pullulanase, an isoamylase, a cellulase, a trehalase, a lipase, a pectinase, a polyesterase, a cutinase, an oxidase, a transferase, a reductase, a hemicellulase, a mannanase, an esterase, an isomerase, a pectinases, a lactase, a peroxidase and a laccase. Proteins of interest may be secreted, glycosylated, and otherwise-modified.

### VI. Use of the modified yeast for increased alcohol production

The present yeast strains and methods of use, thereof, are for increasing the production of alcohol in yeast. Such methods are not limited to a particular fermentation process. The present engineered yeast is expected to be a "drop-in" replacement for convention yeast in any alcohol fermentation facility, whether using raw starch hydrolysis, simultaneous saccharification and fermentation, or other standard variations of conventional ethanol production. While primarily intended for fuel alcohol production, the present yeast can also be used for the production of potable alcohol, including wine and beer.

### VII. Yeast cells suitable for modification

Yeasts are unicellular eukaryotic microorganisms classified as members of the fungus kingdom and include organisms from the phyla Ascomycota and Basidiomycota. Yeast that can be used for alcohol production include, but are not limited to, *Saccharomyces* spp., including *S. cerevisiae,* as well as *Kluyveromyces, Lachancea* and *Schizosaccharomyces* spp. Numerous yeast strains are commercially available, many of which have been selected or genetically engineered for desired characteristics, such as high alcohol production, rapid growth rate, and the like. Some yeast has been genetically engineered to produce heterologous enzymes, such as glucoamylase or α-amylase.

### VII. Substrates and products

Alcohol production from a number of carbohydrate substrates, including but not limited to corn starch, sugar cane, cassava, and molasses, is well known, as are innumerable variations and improvements to enzymatic and chemical conditions and mechanical processes. The present compositions and methods are believed to be fully compatible with such substrates and conditions.

Alcohol fermentation products include organic compound having a hydroxyl functional group (-OH) is bound to a carbon atom. Exemplary alcohols include but are not limited to methanol, ethanol, *n*-propanol, isopropanol, *n*-butanol, isobutanol, *n*-pentanol, 2-pentanol, isopentanol, and higher alcohols. The most commonly made fuel alcohols are ethanol, and butanol.

These and other aspects and embodiments of the present yeast strains and methods will be apparent to the skilled person in view of the present description.

### EXAMPLES

The following examples are intended to further illustrate, but not limit, the described compositions and methods.

### Example 1. Materials and methods

The following protocols were employed unless otherwise specified.

### Liquefact preparation

Liquefact (Ground corn slurry) was prepared by adding 600 ppm of urea, 0.124 SAPU/g ds FERMGEN^{™} 2.5X (acid fungal protease), 0.33 GAU/g ds glucoamylase (a variant *Trichoderma* glucoamylase) and 1.46 SSU/g ds GC626 (*Aspergillus* α-amylase), adjusted to a pH of 4.8.

### AnKom assays

300 µL of concentrated yeast overnight culture was added to each of a number ANKOM bottles filled with 30 g prepared liquefact for a final OD of 0.3. The bottles were then incubated at 32°C with shaking (150 RPM) for 65 hours.

### HPLC analysis

Samples from serum vial and AnKom experiments were collected in Eppendorf tubes by centrifugation for 12 minutes at 14,000 RPM. The supernatants were filtered with 0.2 µM PTFE filters and then used for HPLC (Agilent Technologies 1200 series) analysis with the following conditions: Bio-Rad Aminex HPX-87H columns, running temperature of 55°C. 0.6 ml/min isocratic flow 0.01 N H₂SO₄, 2.5 µL injection volume. Calibration standards were used for quantification of the of acetate, ethanol, glycerol, and glucose. Samples from shake flasks experiments were collected in Eppendorf tubes by centrifugation for 15 minutes at 14,000 RPM. The supernatants were diluted by a factor of 11 using 5 mM H₂SO₄ and incubated for 5 min at 95°C. Following cooling, samples were filtered with 0.2 µM Corning FiltrEX CLS3505 filters and then used for HPLC analysis. 10 µl was injected into an Agilent 1200 series HPLC equipped with a refractive index detector. The separation column used was a Phenomenex Rezex-RFQ Fast Acid H+ (8%) column. The mobile phase was 5 mM H₂SO₄, and the flow rate was 1.0 mL/min at 85 °C. HPLC Calibration Standard Mix from Bion Analytical was used for quantification of the of acetate, ethanol, glycerol, and glucose. Unless otherwise specified, all values are expressed in g/L.

### Example 2. Over-expression of codon-optimized GIS1 in yeast

GIS1, encoded by gene *YDR096W* of Saccharomyces cerevisiae S288c, was codon optimized and synthesized to generate the artificial gene, "GIS1s," shown as SEQ ID NO: 2:

ATGGAAATCAAGCCAGTCGAAGTTATCGACGGTGTTCCAGTCTTCAAGCCATCTATGATGGAAT
TTGCCAATTTTCAATACTTCATTGACGAAATCACCAAGTTTGGTATCGAAAACGGTATTGTCAA
GGTTATTCCTCCCAAGGAATGGCTGGAATTGTTGGAAGGTTCTCCACCTGCTGAATCCTTGAAG
ACTATCCAACTAGATTCTCCAATTCAACAGCAAGCCAAGAGATGGGACAAACACGAAAACGGTG
TCTTTTCCATCGAAAACGAATACGACAACAAGTCTTACAACTTGACACAATGGAAGAATTTGGC
TGAATCCTTGGATTCTAGAATCAGTCAAGGTGACTTCAACGACAAGACCTTAAAGGAAAACTGC
AGAGTCGATTCTCAACAGGATTGTTACGATTTGGCTCAATTACAAATCTTGGAATCCGACTTCT
GGAAGACCATTGCCTTTTCCAAGCCATTCTACGCTGTTGACGAAAACTCTTCCATCTTCCCATA
CGACTTGACTTTATGGAACTTGAACAATTTGCCAGATTCTATCAACTCCAGCAACAGACGTTTG
CTAACTGGTCAATCCAAGTGTATCTTTCCATGGCACTTGGACGAACAAAACAAGTGTTCTATCA
ACTACTTGCACTTCGGTGCTCCAAAGCAATGGTACTCCATTCCATCTGCCAACACCGATCAATT
CTTGAAGATCCTATCCAAGGAACCATCAAGCAACAAGGAAAATTGTCCAGCTTTCATCCGTCAT
CAAAACATCATTACTTCTCCAGACTTTTTGAGAAAGAACAATATCAAGTTCAACAGAGTTGTCC
AATTTCAACATGAATTTATCATTACCTTTCCTTACTGTATGTACTCCGGTTTCAACTACGGTTA
CAACTTTGGCGAATCTATCGAGTTCATCTTAGATCAGCAAGCTGTTGTCAGAAAGCAACCATTG
AAGTGTGGTTGCGGCAACAAGAAAGAAGAGAGAAAGTCTGGTCCATTTTCCAACTTGTCTTACG
ACTCCAACGAAAGCGAACAACGTGGTTCTATTACCGACAACGACAATGATTTGTTTCAAAAGGT
CAGATCCTTCGACGAATTGCTAAACCACTCCTCTCAAGAATTGCAAAACTTGGAAGACAACAAG
AATCCATTGTTTTCCAACATCAATATGAACAGACCACAAAGCTCCTCTTTGAGGTCTACTACAC
CAAACGGTGTCAACCAATTCTTGAACATGAATCAAACTACCATCAGCAGAATTTCCTCTCCATT

GTTATCAAGAATGATGGACTTGTCCAACATCGTGGAACCAACCTTGGACGATCCTGGTTCCAAG
TTCAAGAGAAAGGTTTTGACTCCACAATTACCACAAATGAACATTCCATCCAACTCTTCCAACT
TTGGTACTCCTTCTTTGACCAATACAAACTCCTTGCTATCAAACATCACTGCTACATCTACCAA
TCCATCCACCACTACCAACGGCTCTCAAAACCACAACAATGTCAACGCCAATGGTATCAACACC
TCTGCTGCCGCTTCCATCAACAATAACATTTCCTCTACCAACAATTCTGCCAATAACAGCTCTT
CCAACAATAACGTTTCTACTGTTCCATCTTCCATGATGCACTCTTCCACCTTGAATGGTACTTC
TGGTTTGGGTGGCGACAACGATGACAACATGTTAGCTTTGAGCCTAGCTACCTTGGCCAACAGT
GCTACTGCTTCTCCAAGATTGACCTTACCACCTTTGTCTTCACCAATGAATCCCAACGGTCACA
CTTCCTACAACGGTAACATGATGAACAATAACTCTGGTAACGGTTCCAACGGTAGCAACTCTTA
CTCCAATGGTGTCACCACTGCTGCCGCTACCACTACATCTGCTCCACACAACTTGTCCATCGTT
TCTCCAAACCCTACCTACAGTCCAAATCCCTTGTCTCTATACTTGACCAACTCCAAGAATCCAT
TGAACTCTGGTTTGGCTCCATTATCTCCTTCCACTTCTAACATTCCATTCTTGAAGAGAAACAA
TGTCGTTACCCTAAACATCTCCAGAGAAGCCTCCAAGTCTCCAATCTCTTCCTTTGTCAACGAC
TACCGTTCTCCATTGGGTGTTTCCAATCCATTGATGTACTCTTCCACTATCAACGATTACTCCA
ACGGTACTGGTATCCGTCAAAACAGCAACAACATCAATCCATTGGACGCTGGTCCATCTTTTTC
TCCTTTGCACAAGAAACCAAAGATTTTGAACGGCAATGACAACTCCAATTTGGACAGCAACAAT
TTCGATTACTCTTTTACTGGTAACAAGCAAGAATCCAATCCATCTATCTTGAACAACAATACCA
ACAATAACGACAACTACAGAACTTCTTCCATGAACAACAATGGTAACAATTACCAAGCTCACTC
TTCCAAGTTTGGTGAAAACGAAGTCATCATGTCCGATCACGGTAAGATTTACATCTGTAGAGAA
TGTAACAGACAATTTTCCTCTGGTCACCATCTAACCAGACACAAAAAGTCAGTTCATTCTGGTG
AAAAGCCACACTCTTGTCCAAGATGTGGTAAAAGATTCAAGAGAAGAGATCATGTCTTGCAACA
CTTGAACAAGAAAATCCCATGCACTCAAGAAATGGAAAACACCAAGTTGGCTGAATCTTAA

The ADR1 promoter (represented by SEQ ID NO: 3) was operably-linked to the 5'-end of the codon-optimized GIS1s coding sequence to control expression:

AATAGAGTATGATTATTTTTTTTATATTTTTTTTTTTTGGAAAACAAAATTCTTATAGTAAAGT
AAGGAATAGTAGCAGAATATTTTTCTGAAGTGTTTATAATAAAGGGAGAACCGGGAAAGTAGCA
AAATGATTGGTTAATTTATGCAAATCAATCTTATACTTCCAACGAATAAGAGGGAGTATATCAA
AACAGAGTAACAATAAACTTTTGCTATGACACCTTTTCTTTCTTTCAAAGATAAAAGAATAAGG
CTTTTCTATAGTAGTCGAGCAAATGTTGGAATAAGGAGGTATGGAATTTTGAAAATAGCCTGAG
AAATTCAGATCAATGATATATAACTGTTGTCTTCAAAAGGTCTTCAAGGGAAGAAAATTTCCGA
TCGGAAGTCCAGAATAAATACCACAAGTATAGCACAATAAACAACAGCACAACAACAATAATAA
CAACACCTGTAGCGAAAACGTTTTCCTATTTTTAAGGCGTTGTTCTTTGAAAACCTGTGGCGAA
GTAAAACATGAAAACAAATGAAAACACCGCCAAACCAAGAAAAGAACAACGAAAAAATATCACT
TTTATTTAGTTCACAACGGCTAACTATCGACGTTCACCCTTCCTCAGTCTATCACATCGTCCTT

AGCTCGAACAACGCCGATAGGCATCAAGTTACATTGAGCTTTACTGCACGTTCCCGCATGATGC
CATTGACTAGGGCCCGCCCTTTCGGCAATCATTCTAGCATGTTCCGCATGTTC

The Eno2 terminator (*YHR174W* locus; SEQ ID NO: 4) was operably linked to the 3'-end of the codon-optimized GIS1s coding sequence to complete the
ADR1Pro::GIS1ss::Ebo2Ter expression cassette:

GTGCACCTTTTTTTTCTCCTTCCAGTGCATTATGCAATAGACAGCACGAGTCTTTGAAAAAGTA
ACTTATAAAACTGTATCAATTTTTAAACCTAAATAGATTCATAAACTATTCGTTAATATAAAGT
GTTCTAAACTATGATGAAAAAATAAGCAGAAAAGACTAATAATTCTTAGTTAAAAGCACTTTAC
AACTTGTCACCGTGGTGGAAGTTTTCACCG

The amino acid sequenc of the GIS1 polypeptide from *Saccharomyces cerevisiae* S288c (Genbank Accession No. NP_010381.1) is shown, below, as SEQ ID NO: 5:

This expression cassette was amplified using primers with appropriate flanking sequences to promote insertion at the PCT7 site (*YHR076W*) and transformed into either (i) FERMAX^{™} Gold (Martrex Inc., Minnesota, USA; herein abbreviated, "FG"), a well-known fermentation yeast used in the grain ethanol industry, or (ii) FG-PKL, engineered FG yeast having a heterologous phosphoketolase (PKL) pathway involving the expression of phosphoketolase (PKL), phosphotransacetylase (PTA) and acetylating acetyl dehydrogenase (AADH) as described in WO2015148272 (Miasnikov et al.)*.* The expected insertion of the GIS1 expression cassette into the two parental strains was confirmed by PCR.

### Example 3. Alcohol production using yeast that over-express GIS1s

Strains over-expressing GIS1 were tested in an Ankom assay, containing 30 g liquefact. Fermentations were performed at 32°C for 55 hours. Samples from the end of fermentation were analyzed by HPLC. The results are summarized in Tables 1 and 2.

**Table 1. HPLC results from FG and FG-GIS1s strains**

| **Strain features** | **Glycerol (g/L)** | **Acetate (g/L)** | **Glucose (g/L)** | **Ethanol (g/L)** | **Ethanol increase (%)** |
|---|---|---|---|---|---|
| FG | 13.06 | 0.91 | 0.33 | 143.44 | -0- |
| FG-GIS1s | 12.99 | 0.85 | 0.40 | 145.42 | 1.4 |

**Table 2. HPLC results from FG-PKL and FG-PKL-GIS1s**

| **Strain features** | **Glycerol (g/L)** | **Acetate (g/L)** | **Glucose (g/L)** | **Ethanol (g/L)** | **Ethanol increase (%)** |
|---|---|---|---|---|---|
| FG-PKL | 12.53 | 1.55 | 0.70 | 147.27 | -0- |
| FG-PKL-GIS1s | 12.01 | 1.72 | 0.26 | 148.90 | 1.1 |

Over-expression of GIS1 resulted in 1.4% increase of ethanol production in FG yeast, which is recognized as a robust, high-ethanol-producing yeast for the fuel ethanol industry, while not being a genetically engineered organism. Over-expression of GIS1s resulted 1.1% increase of ethanol production in FG yeast engineered to have an exogenous PKL pathway. These results again demonstrate that GIS1 over-expression is beneficial for increasing ethanol production.

## Claims

1. A method for increasing the production of ethanol in yeast cells grown on a carbohydrate substrate, comprising: introducing into parental yeast cells a genetic alteration that causes the modified cells to produce an increased amount of GIS1 polypeptides compared to the parental cells, wherein the modified cells produce an increased amount of ethanol compared to otherwise identical parental cells under equivalent fermentation conditions.

2. The method of claim 1, wherein the genetic alteration comprises introduction into the parental cells of a nucleic acid capable of directing the expression of GIS1 to a level greater than that of the parental cell grown under equivalent conditions.

3. The method of claim 1 or claim 2, wherein the genetic alteration comprises the introduction of an exogenous *YDR096W* gene.

4. The method of claim 1 or claim 2 wherein the genetic alteration comprises the introduction of a stronger promoter in an endogenous *YDR096W* gene.

5. The method of claim 1 or claim 2, wherein the genetic alteration comprises introduction of an expression cassette for expressing GIS1.

6. The method of any of claims 1-5, wherein the cells further comprise one or more genes of the phosphoketolase pathway.

7. The method of claim 6, wherein the genes of the phosphoketolase pathway are selected from the group consisting of phosphoketolase, phosphotransacetylase and acetylating acetyl dehydrogenase.

8. The method of any of claims 1-7, wherein the amount of increase in the expression of GIS1 is at least 10-fold higher compared to the level of expression in parental cells grown under equivalent conditions, based on GIS1-encoding mRNA expression.

9. The method of any of claims 1-8, wherein the cells further comprise an exogenous gene encoding a carbohydrate processing enzyme.

10. The method of any of claims 1-9, wherein the cells further comprise an alteration in the glycerol pathway and/or the acetyl-CoA pathway.

11. The method of any of claims 1-10, wherein the cells further comprise an alternative pathway for making ethanol.

12. The method of any of claims 1-11, wherein the cells are of a *Saccharomyces* spp.

## Patentansprüche

1. Verfahren zur Steigerung der Ethanolproduktion von Hefezellen, die auf einem Kohlenhydratsubstrat angezüchtet werden, Folgendes umfassend: Einführen einer gentechnischen Veränderung in die Ausgangshefezellen, wobei diese bewirkt, dass die veränderten Zellen eine Menge an GIS1-Polypeptiden produzieren, die im Vergleich zu den Ausgangszellen erhöht ist, wobei die veränderten Zellen im Vergleich zu Ausgangszellen, die ansonsten vollkommen gleichartig sind, unter gleichwertigen Fermentationsbedingungen eine erhöhte Menge an Ethanol produzieren.

2. Verfahren nach Anspruch 1, wobei die gentechnische Veränderung das Einführen einer Nukleinsäure in die Ausgangszellen umfasst, die dazu befähigt ist, die Expression von GIS1 auf ein Niveau zu bringen, das höher als dasjenige der Ausgangszellen ist, wenn diese unter gleichwertigen Bedingungen angezüchtet werden.

3. Verfahren nach Anspruch 1 oder Anspruch 2, wobei die gentechnische Veränderung das Einführen eines exogenen *YDR096W-Gens* umfasst.

4. Verfahren nach Anspruch 1 oder Anspruch 2, wobei die gentechnische Veränderung das Einführen eines stärkeren Promotors in ein exogenes *YDR096W*-Gen umfasst.

5. Verfahren nach Anspruch 1 oder Anspruch 2, wobei die gentechnische Veränderung das Einführen einer Expressionskassette für die Expression von GIS1 umfasst.

6. Verfahren nach einem beliebigen der Ansprüche 1 bis 5, wobei die Zellen weiterhin ein oder mehrere Gene des Phosphoketolase-Stoffwechselwegs umfassen.

7. Verfahren nach Anspruch 6, wobei die Gene des Phosphoketolase-Stoffwechselwegs aus der Gruppe ausgewählt sind, die aus Phosphoketolase, Phosphotransacetylase und acetylierender Acetyldehydrogenase besteht.

8. Verfahren nach einem beliebigen der Ansprüche 1 bis 7, wobei das Ausmaß der Steigerung der Expression von GIS1 mindestens dem 10-fachen entspricht, im Vergleich zu dem Expressionsniveau bei Ausgangszellen, welche unter gleichwertigen Bedingungen angezüchtet werden, unter Bezugnahme auf die Expression der mRNA, welche für GIS1 codiert.

9. Verfahren nach einem beliebigen der Ansprüche 1 bis 8, wobei die Zellen weiterhin ein exogenes Gen umfassen, welches für ein kohlenhydratverarbeitendes Enzym codiert.

10. Verfahren nach einem beliebigen der Ansprüche 1 bis 9, wobei die Zellen weiterhin eine Veränderung im Glycerin-Stoffwechselweg und/oder im Acetyl-CoA-Stoffwechselweg umfassen.

11. Verfahren nach einem beliebigen der Ansprüche 1 bis 10, wobei die Zellen weiterhin einen alternativen Stoffwechselweg zur Herstellung von Ethanol umfassen.

12. Verfahren nach einem beliebigen der Ansprüche 1 bis 11, wobei die Zellen einer *Saccharomyces* spp. angehören.

## Revendications

1. Procédé pour augmenter la production d'éthanol dans des cellules de levure qui ont été cultivées sur un substrat de glucide, comprenant : l'introduction dans des cellules de levure parentes d'une altération génétique qui amène les cellules modifiées à produire une quantité augmentée de polypeptides GIS1 par comparaison avec les cellules parentes, les cellules modifiées produisant une quantité augmentée d'éthanol par comparaison avec des cellules parentes par ailleurs identiques dans des conditions de fermentation équivalentes.

2. Procédé selon la revendication 1, l'altération génétique comprenant une introduction dans les cellules parentes d'un acide nucléique capable de diriger l'expression de GIS1 jusqu'à un niveau supérieur à celui de la cellule parente cultivée dans des conditions équivalentes.

3. Procédé selon la revendication 1 ou la revendication 2, l'altération génétique comprenant l'introduction d'un gène *YDR096W* exogène.

4. Procédé selon la revendication 1 ou la revendication 2, l'altération génétique comprenant l'introduction d'un promoteur plus fort dans un gène *YDR096W* endogène.

5. Procédé selon la revendication 1 ou la revendication 2, l'altération génétique comprenant l'introduction d'une cassette d'expression pour exprimer GIS1.

6. Procédé selon l'une quelconque des revendications 1 à 5, les cellules comprenant en outre un ou plusieurs gènes de la voie de la phosphokétolase.

7. Procédé selon la revendication 6, les gènes de la voie de la phosphokétolase étant choisis dans le groupe constitué par la phosphokétolase, la phosphotransacétylase et l'acétyldéshydrogénase acétylante.

8. Procédé selon l'une quelconque des revendications 1 à 7, la quantité d'augmentation de l'expression de GIS1 étant au moins 10 fois supérieure par comparaison avec le niveau d'expression dans des cellules parentes cultivées dans des conditions équivalentes, sur la base de l'expression d'ARNm codant pour GIS1.

9. Procédé selon l'une quelconque des revendications 1 à 8, les cellules comprenant en outre un gène exogène codant pour une enzyme de transformation de glucide.

10. Procédé selon l'une quelconque des revendications 1 à 9, les cellules comprenant en outre une altération dans la voie du glycérol et/ou la voie de l'acétyl-CoA.

11. Procédé selon l'une quelconque des revendications 1 à 10, les cellules comprenant en outre une voie alternative pour préparer de l'éthanol.

12. Procédé selon l'une quelconque des revendications 1 à 11, les cellules étant d'une espèce de *Saccharomyces.*
